# EUROPEAN PATENT SPECIFICATION

(11) **EP 2 862 850 B1**
(45) Date of publication and mention of the grant of the patent: **29.11.2017**
(21) Application number: 13804254.4
(22) Date of filing: 14.06.2013
(51) Int. Cl.: C07C 49/167, C07C 43/12, C07C 21/18

(54) **PREPARATION METHOD FOR PERFLUORO-2-METHYL-3-PENTANONE AND INTERMEDIATE**
VERFAHREN ZUR HERSTELLUNG VON PERFLUOR-2-METHYL-3-PENTANON UND ZWISCHENPRODUKT
PROCÉDÉ DE PRÉPARATION DE PERFLUORO-2-MÉTHYL-3-PENTANONE ET PRODUIT INTERMÉDIAIRE

(30) Priority: 15.06.2012 CN 201210197034; 21.06.2012 CN 201210205884; 21.06.2012 CN 201210205922
(43) Date of publication of application: 22.04.2015
(73) Proprietor: Sinochem Lantian Co., Ltd., Hangzhou, Zhejiang 310051 (CN); Zhejiang Lantian Environmental Protection Hi-Tech Co. Ltd., Zhejiang 310018 (CN)
(72) Inventor: NI, Hang, Hangzhou Zhejiang 310023 (CN); ZHANG, Jianjun, Hangzhou Zhejiang 310023 (CN); BAI, Zhanqi, Hangzhou Zhejiang 310023 (CN); RAN, Deqiang, Hangzhou Zhejiang 310023 (CN); PAN, Yan, Hangzhou Zhejiang 310023 (CN); FANG, Xiaoqing, Hangzhou Zhejiang 310023 (CN)
(74) Representative: Kransell & Wennborg KB
(86) International application number: PCT/CN2013/077217
(87) International publication number: WO 2013/185626

(56) References cited:
- GB-A- 1 511 470
- US-A- 3 213 134
- US-A- 5 457 238
- ZAPEVALOV, A. YA. ET AL.: 'Oxides of hexafluoropropylene dimers' IZV. AKAD. NAUK SSSR, SER. KHIM. vol. 12, 1979, pages 2812 - 2815, XP008175521
- CHEPIK, S. D. ET AL.: 'Electrophilic isomerization of fluoroaliphatic oxygen-containing compounds.' IZV. AKAD. NAUK SSSR, SER. KHIM. vol. 11, 1991, pages 2611 - 2618, XP055177344

## Description

### FIELD OF THE INVENTION

The prevention relates to a preparation method for perfluoro-2-methyl-3-pentanone and intermediates.

### BACKGROUND OF THE INVENTION

Perfluoro-2-methyl-3-pentanone is an important compound and can be used as cleaning agent, solvent and extinguishant. Its Ozone Depletion Potential (ODP) is zero, and its Global Warming Potential (GWP) is one. It is a substitute for a new generation of Ozone Depleting Substances (ODS), and can be used as a substitute for Halon 1301 as the extinguishant.

There are mainly two kinds of preparation methods for perfluoro-2-methyl-3-pentanone: (1) a oxidation rearrangement method for hexafluoropropylene dimer, and (2) an addition method for perfluoro propionyl fluoride and hexafluoropropylene.

A method for preparing perfluoro-2-methyl-3-pentanone by the addition of perfluoropropionyl fluoride and hexafluoropropylene can refer to American Patent US6630075 by 3M Company. This patent describes a method for preparing perfluoro-2-methyl-3-pentanone by addition reaction of perfluoropropionyl fluoride and hexafluoropropylene in a pressure vessel. As the method requires pressurized operation and perfluoropropionyl fluoride has strong corrosive, the vessel shall have high material requirements.

Hexafluoropropylene dimer is prepared before perfluoro-2-methyl-3-pentanone is prepared through the oxidation and rearrangement of hexafluoropropylene dimer. Hexafluoropropylene dimer includes two isomers which are perfluoro-4-methyl-2-pentene and perfluoro-2-methyl-2-pentene, respectively. Perfluoro-2-methyl-2-pentene may be oxidized into corresponding epoxide (Perfluoro-2,3-epoxy-2-methyl pentane). Then, perfluoro-2,3-epoxy-2-methyl pentane is converted into perfluoro-2-methyl-3-pentanone through catalysis and rearrangement.

There are mainly two preparation methods for perfluoro-2-methyl-2-pentene, that is, it is generated by the oligomerization of hexafluoropropene or the isomerization of perfluoro-4-methyl-2-pentene as raw material. The oligomerization method includes a gas-phase oligomerization method and a liquid-phase oligomerization method. The gas-phase oligomerization method, which is described in Patent US4377717, Patent US4296265 and Document J. Org. Chem., 30, 3524 (1965), uses hexafluoropropylene as raw material, does not need solvent and uses as catalysts metal fluoride, activated carbon and metal fluoride which are deposited on activated carbon. This method has characteristics of high temperature and pressurization, and requires high quality of equipment. Its products have lower contents of perfluoro-2-methyl-2-pentene and mainly have perfluoro-4-methyl-2-pentene or hexafluoropropylene trimer. According to the liquid-phase oligomerization method in China Patent US4042638, China Patent CN93121609 and Document Fluorine Chem., 9, 94 (1977), the products have oligomerization reaction under the action of polar solvent (N, N'- dimethylformamide, dimethyl sulfoxide, acetonitrile, etc.) and the catalyst (potassium fluoride, potassium sulfide nitrile, nitrile, potassium, ammonium fluoride, etc.), and are controlled and generated by changing solvent and catalyst. Crown ethers may be added to improve the solubility of the catalyst in the system, speeding up the reaction rate.The method is generally used for preparing hexafluoropropylene dimer. Its main product is perfluoro-4-methyl-2-pentene, of which the content is generally more than 90%.

Currently, perfluoro-2-methyl-2-pentene is prepared by using perfluoro-4-methyl-2-pentene as raw material and through isomerization reaction, which has industrial significance. According to the preparation method in Patent GB1511470, perfluoro-2-methyl-2-pentene is prepared by catalysis of potassium fluoride and 18-crown-6. The method uses acetonitrile as solvent and improves reaction results by changing the ratio and amount of the catalyst and reaction temperature. As to the method, the suitable reaction temperature is 40°C; the amount of catalyst is 5 mol%; the reaction time lasts 3 hours, and the conversion rate is 99.3%.The method shall be carried out in a Kjeldahl flask. The substance shall be pre-mixed before feeding. Therefore, the reaction time is longer.

Once there is perfluoro-2-methyl-2-pentene or perfluoro-4-methyl-2-pentene, perfluoro-2, 3-epoxy-2-methyl-pentyl alkyl or perfluoro-2, 3-epoxy-4-methylpentane may be obtained through appropriate oxidation.

For example, according to the preparation method in Patent SU666176, Document Zhurnal Organicheskoi Khimii., 22(1), 93 (1986), Document Izvestiya Akademii Nauk SSSR, Seriya Khimicheskaya., 12, 2812 (1979) and Document Izvestiya Akademii Nauk SSSR, Seriya Khimicheskaya., 11, 2509-12, perfluoro-2-methyl-3-pentanone is prepared by using perfluoro-2-methyl-2-pentene as raw material. As to the method, sodium hypochlorite, of which the volume is five times as big as that of reaction substrate, is taken as oxidant; dioxane or acetonitrile and water are taken as solvent; the reaction time lasts one hour; and Yield are 68%, 93%, 94% and 93%, respectively. As to the method, perfluoro-2,3-epoxy-2-methyl pentane may be obtained with homemade sodium hypochlorite as the oxidant (the concentration homemade sodium hypochlorite is usually higher than that is commercially available) and pH which is controlled to be between 9-11. Document Tetrahedron Letters., 44(43), 7961 (2002) describes an oxidation method for triethylamine oxide.As to the method, triethylamine oxide is taken as oxidant; N, N'- dimethylformamide is taken as solvent; the reaction time lasts one hour at the temperature of -30°C, and yield is 98%. The method has the advantages of high oxidation efficiency and short reaction time, but has the disadvantages of difficult oxide preparation, greater risk, lower reaction temperature, and high cost in mass production.

Document Zhurnal Organicheskoi Khimii., 22(1), 93 (1986), Document Izvestiya Akademii Nauk SSSR, Seriya Khimicheskaya., 12, 2812 (1979) (also referred to as ZAPEVALOV, A. VA. ET AL.: "Oxides of hexafluoropropylene dimers", IZV. AKAO. NAUK SSSR, SER. KHIM., vol. 12,1979, pages 2812-2815), Document Izvestiya Akademii Nauk SSSR, Seriya Khimicheskaya., 11, 2509-12(1979) and Document Izvestiya Akademii Nauk SSSR, Seriya Khimicheskaya., 11, 2611-18(1991) (also referred to as CHEPIK, S. O. ET AL.: "Electrophilic isomerization of fluoroaliphatic oxygen-containing compounds.", IZV. AKAO. NAUK SSSR, SER. KHIM., vol. 11, 1991, pages 2611-2618) describe a preparation method for fluoride or triethylamine catalysis. As to the method, perfluoro-2,3-epoxy-2-methyl pentane or perfluoro-2,3-epoxy-4-methylpentane is taken as substrate; sodium fluoride, potassium fluoride, cesium fluoride, antimony pentafluoride or triethylamine is taken as catalyst; glyme, diglyme or tetraglyme is taken as solvent; reaction temperature is 20-250°C; reaction time lasts 2-10 hours, and the highest conversion rate of perfluoro-2-methyl-3-pentanone is up to 94%.In most cases, the method has mild reaction conditions; however, the reaction temperature is up to 250°C when antimony pentafluoride is taken as catalyst; there are more by-products, such as perfluoro-4-methyl-2-pentanone or perfluoro -2, 4-dimethyl-1-oxa-cyclopentane, and selectivity is not high.

### SUMMARY OF THE INVENTION

The invention provides a preparation method for perfluoro-2, 3-epoxy-2-methyl-pentane and perfluoro-2-methyl-3-pentanone, with the advantages of mild reaction conditions, fast reaction rate, and high selectivity.

In order to achieve the above objective of the invention, the invention adopts any one of the following technical solutions:
According to one aspect, the invention provides a preparation method for perfluoro-2-methyl-3-pentanone.
According to the method of the invention, in the presence of fluoride salts and ether compounds, perfluoro-2, 3-epoxide-2-methyl pentane is converted into perfluoro-2-methyl-3-pentanone by a catalytic rearrangement reaction at the reaction temperature of 10∼70 °C;

In any one of the above technical solutions, the fluoride salt may be selected from one of or the combination of two or more than three of lithium fluoride, sodium fluoride, potassium fluoride, cesium fluoride and aluminum fluoride;

In any one of the above technical solutions, the ether compound may be selected from one of or the combination of two or more than three of diethyl ether, sulfolane, 15-crown-5 and 18-crown-6;

In any one of the above technical solutions, the molar ratio of the ether compound and the fluoride salts is 0.5∼ 5.0:1. (that is to say, the proportion shall be within 0.5:1 to 5.0:1. Note: the numbers at the two ends and any number between the two ends shall fall within any number scope of the invention, although these numbers between the two ends are not particularly disclosed separately, they shall be regarded as have described separately.);

In any one of the above technical solutions, the molar ratio of the fluoride salts and perfluoro-2,3-epoxy-2-methyl pentane is 0.02∼0.5: 1.

The invention provides a preparation method for perfluoro-2-methyl-3-pentanone where catalyst and cocatalyst are used for facilitating rearrangement reaction, wherein fluoride salt is taken as catalyst, ether compounds are taken as cocatalysts. fluoride salt is preferably selected from one, two or three of potassium fluoride, cesium fluoride and aluminum fluoride. The ether compound is preferably selected from one, two or three of sulfolane, 15-crown-5 and 18-crown-6.The amount of catalyst and cocatalyst affects the reaction,the molar ratio of the fluoride salts and perfluoro-2,3-epoxy-2-methyl pentane is preferably 0.05∼0.35: 1, and further preferably 0.07∼ 0.30:1; the molar ratio of the ether compound and the fluoride salts is preferably 0.75∼3.0:1, and further preferably 1.0∼2.5:1.

The reaction temperature in the invention has certain effect on the conversion rate of the rearrangement reaction, The increase in temperature facilitates the rise in conversion rate but causes the decrease in selectivity. The suitable reaction temperature is 10 ∼ 70 °C , preferably 30∼60 °C and further preferably 35∼55 °C.

Any one of the above technical solutions for preparing perfluoro - 2 - methyl - 3 - pentanone may further include any one of preparation methods for perfluoro-2,3-epoxy-2-methyl pentane as described below.

According to one aspect, the invention provides a preparation method for perfluoro-2,3-epoxy-2-methyl pentane, the prepared perfluoro-2,3-epoxy-2-methyl pentane may be used for any one of the above technical solutions for preparing perfluoro-2-methyl-3-pentanone. As to the technical solution in the invention for preparing perfluoro-2,3-epoxy-2-methyl pentane, with amino compound as epoxidation catalyst, in the presence of aprotic solvent, at the reaction temperature of -5∼35 °C, perfluoro-2-methyl-2-pentene and oxidant (for example, sodium hypochlorite) are reacted and converted into the perfluoro-2,3-epoxy-2-methyl pentane;

In any one of the above technical solutions, the amino compound may be selected from one of or the combination of two or more three of trimethylamine, diethylamine, triethylamine, urea and tetramethyl urea;

In any one of the above technical solutions, the oxidant may be selected from hypochlorous acid or hypochlorite (for example, sodium hypochlorite, calcium hypochlorite, etc.).

In any one of the above technical solutions, the molar ratio of the sodium hypochlorite and perfluoro-2-methyl-2-pentene is 1∼2: 1;

In any one of the above technical solutions, the molar ratio of the epoxidation catalyst and perfluoro-2-methyl-2-pentene is 0.02∼0.30: 1.

The oxidant used in the invention may be calcium hypochlorite solution and sodium hypochlorite solution (for example, 10% sodium hypochlorite solution) which are commercially available, and certainly may be prepared by the user according to requirements, too. the molar ratio of the oxidant and perfluoro-2-methyl-2-pentene is preferably 1.0∼1.6: 1, and further preferably 1.1.0∼1.4:1.

The epoxidation catalyst used in the invention is preferably selected from one, two or three of trimethylamine, diethylamine and urea, and further preferably trimethylamine and/or urea. the addition amount of epoxidation catalyst has effects on the conversion rate of epoxidation reaction, the molar ratio of the epoxidation catalyst and perfluoro-2-methyl-2-pentene is preferably 0.05∼0.20: 1, and further preferably 0.07∼0.15:1.

In the invention, the reaction temperature has effects on the conversion rate of epoxidation reaction. The low temperature facilitates the rise in selectivity, but the rise in the reaction temperature facilitates the increases in the reaction rate, and causes the oxidant (for example, sodium hypochlorite) to be decomposed too rapid. therefore, the temperature of general epoxidation reaction is -5∼35 °C, preferable 5∼20 °C, and further preferably 10∼15 °C.

In the technical solution of the invention, amino compounds and oxidants may react in the reaction solution and generate epoxidation catalyst, Once epoxidation catalyst generated in situ is generated, it and perfluoro-2-methyl-2-pentene react and generate epoxy products, which has the advantages of high efficiency and high stability. Therefore, the reaction may be carried out under more convenient conditions (for example, nearly room temperature).

In any one of the above technical solutions, the needed perfluoro-2-methyl-2-pentene may be prepared by any one of the following methods. So, any one of the above technical solutions may otherwise include any one of the following preparation methods.

According to another aspect, the invention provides a preparation method for perfluoro-2-methyl-2-pentene with the advantages of simplicity and low requirements for equipment. The technical solution of the invention is to provide a method for preparing perfluoro-2-methyl-2-pentene through the catalytic isomerization of the perfluoro-4-methyl-2-pentene, the perfluoro-2-methyl-2-pentene is generated by the isomerization of perfluoro-4-methyl-2-pentene in aprotic polar solvent under the action of catalyst at the temperature of 40 ∼ 80 °C,
in any one of the above technical solutions, the catalyst consists of procatalyst and cocatalyst,the procatalyst is selected from one of or the combination of two or more than three of lithium fluoride, sodium fluoride, potassium fluoride, cesium fluoride or aluminum fluoride;
in any one of the above technical solutions, the cocatalyst is selected from one of or the combination of two or more than three of diethyl ether, sulfolane, 15-crowns-5 or 18- crowns -6;
in any one of the above technical solutions, the molar ratio of the procatalyst and the cocatalyst is 1∼0.1:1;
in any one of the above technical solutions, the molar ratio of the procatalyst and perfluoro-4-methyl-2-pentene is 0.05∼0.5: 1;
in any one of the above technical solutions, the molar ratio of the co-catalyst and perfluoro-4-methyl-2-pentene is 0.05∼0.5: 1;
in any one of the above technical solutions, the aprotic solvent is selected from one of or the combination of two or more than three of glyme, diglyme (DG for short) acetonitrile or N, N'- dimethylformamide.
in any one of the above technical solutions, the molar ratio of procatalyst and cocatalyst may be greater than, less than or equal to 1. However, during the experiment process, the inventor finds that when the molar ratio of procatalyst and cocatalyst is less than or equal to 1, the conversion rate and the molar ratio of main catalyst and co-catalyst are proportional. Therefore, in any one of the above technical solutions, the molar ratio of procatalyst and cocatalyst is 0.8∼0.2:1;
in any one of the above technical solutions, the above reaction temperature is preferably 40∼70 °C.
in any one of the above technical solutions, the above procatalyst is preferably potassium fluoride and / or cesium fluoride, and cocatalyst is preferably 18-crown-6 ether and / or sulfolane.

In any one of the above technical solutions, the molar ratio of the procatalyst and perfluoro-4-methyl-2-pentene is preferably 0.1∼0.2: 1.

In any one of the above technical solutions, the molar ratio of the cocatalyst and perfluoro-4-methyl-2-pentene is preferably 0.15∼0.35: 1.

In any one of the above technical solutions, the above aprotic solvent is preferably diethylene glycol dimethyl ether and / or acetonitrile.

Compared with the prior art, the preparation method of the invention has the characteristics of simple operation, no high temperature and pressure, fast response and good safety.

### DETAILED DESCRIPTION OF THE EMBODIMENTS

The invention is further described with combination with the examples, but is not limited to these examples. The person skilled in the art shall note that the invention covers all of alternative solutions, improvement solutions and equivalent solutions which may be included within the scope of claims.

### Example 1

158 g of perfluoro-2,3-epoxy-2-methyl pentane, 2.1 g of sodium fluoride, 6 g of sulfolane and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 57.9%.

### Example 2

158 g of perfluoro-2,3-epoxy-2-methyl pentane, 2.9 g of potassium fluoride, 6 g of sulfolane and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 65.6%.

### Example 3

158 g of perfluoro-2,3-epoxy-2-methyl pentane, 7.6 g of cesium fluoride, 6 g of sulfolane and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 70.1%.

### Example 4

158 g of perfluoro-2,3-epoxy-2-methyl pentane, 4.2g of aluminum fluoride, 6 g of sulfolane and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 75.0%.

### Example 5

158 g of perfluoro-2,3-epoxy-2-methyl pentane, 2.1g of sodium fluoride, 11 g of 15-crown-5 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 83.8%.

### Example 6

158 g of perfluoro-2,3-epoxy-2-methyl pentane, 4.2g of aluminum fluoride, 11 g of 15-crown-5 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 82.1%.

### Example 7

158 g of perfluoro-2,3-epoxy-2-methyl pentane, 2.1g of sodium fluoride, 13.2g of 18-crown-6 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 89.3%.

### Example 8

158 g of perfluoro-2,3-epoxy-2-methyl pentane, 2.9g of potassium fluoride, 13.2g of 18-crown-6 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 90.5%.

### Example 9

158 g of perfluoro-2,3-epoxy-2-methyl pentane, 4.2g of aluminum fluoride, 13.2 g of 18-crown-6 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 67.3%.

**Table 1**

| Examples | Catalysts | Additives | Mass of Catalysts/G | Mass of Additives/G | Conversion Rate |
|---|---|---|---|---|---|
| 1 | Sodium Fluoride | Sulfolane | 2.1 | 6 | 57.9% |
| 2 | Potassium Fluoride | Sulfolane | 2.9 | 6 | 65.6% |
| 3 | Caesium Fluoride | Sulfolane | 7.6 | 6 | 70.1% |
| 4 | Aluminium Fluoride | Sulfolane | 4.2 | 6 | 75.0% |
| 5 | Sodium Fluoride | 15-Crown-5 | 2.1 | 11 | 83.8% |
| 6 | Aluminium Fluoride | 15-Crown-5 | 4.2 | 11 | 82.1% |
| 7 | Sodium Fluoride | 18-Crown-6 | 2.1 | 13.2 | 89.3% |
| 8 | Potassium Fluoride | 18-Crown-6 | 2.9 | 13.2 | 90.5% |
| 9 | Aluminium Fluoride | 18-Crown-6 | 4.2 | 13.2 | 67.3% |

### Example 10

A reaction device and operation conditions are the same as those of Example 6, but the feeding quantity of catalysts and additives is changed. 158 g of perfluoro-2,3-epoxy-2-methyl pentane, 2.1g of aluminum fluoride, 5.5g of 15-crown-5 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 60.8%.

### Example 11

A reaction device and operation conditions are the same as those of Example 6, but the feeding quantity of catalysts and additives is changed. 158 g of perfluoro-2,3-epoxy-2-methyl pentane, 6.3g of aluminum fluoride, 16.5g of 15-crown-5 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 95.7%.

### Example 12

A reaction device and operation conditions are the same as those of Example 6, but the feeding quantity of catalysts and additives is changed. 158 g of perfluoro-2,3-epoxy-2-methyl pentane, 12.6g of aluminum fluoride, 33.0g of 15-crown-5 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 95.9%.

### Example 13

A reaction device and operation conditions are the same as those of Example 6, but the feeding quantity of catalysts and additives is changed. 158 g of perfluoro-2,3-epoxy-2-methyl pentane, 6.3g of aluminum fluoride, 33.0g of 15-crown-5 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 95.1%.

### Example 14

A reaction device and operation conditions are the same as those of Example 6, but the feeding quantity of catalysts and additives is changed. 158 g of perfluoro-2,3-epoxy-2-methyl pentane, 6.3g of aluminum fluoride, 49.5g of 15-crown-5 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 87.9%.

**Table 2**

| Examples | Mass of Additives/g | Mass of Additives/g | Conversion Rate |
|---|---|---|---|
| 10 | 2.1 | 5.5 | 60.8% |
| 11 | 6.3 | 16.5 | 95.7% |
| 12 | 12.6 | 33.0 | 95.9% |
| 13 | 6.3 | 33.0 | 95.1% |
| 14 | 6.3 | 49.5 | 87.9% |

### Example 15

A reaction device and operation conditions are the same as those of Example 8, but the feeding quantity of catalysts and additives is changed. 158 g of perfluoro-2,3-epoxy-2-methyl pentane, 1.5g of potassium fluoride, 13.2g of 18-crown-6 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 79.5%.

### Example 16

A reaction device and operation conditions are the same as those of Example 8, but the feeding quantity of catalysts and additives is changed. 158 g of perfluoro-2,3-epoxy-2-methyl pentane, 2.9g of potassium fluoride, 6.6g of 18-crown-6 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 87.4%.

### Example 17

A reaction device and operation conditions are the same as those of Example 8, but the feeding quantity of catalysts and additives is changed. 158 g of perfluoro-2,3-epoxy-2-methyl pentane, 4.5g of potassium fluoride, 19.8g of 18-crown-6 and 100 mL of diglyme are added into a 500 mL three-neck flask equipped with a reflux condensing tube and a mechanical stirring device, respectively. The temperature is raised to be 30 °C after stir starts. After 3 hours of reaction, the reaction product of perfluoro-2-methyl-3-pentanone is analyzed by gas chromatography, the conversion rate of perfluoro-2,3-epoxy-2-methyl pentane is 99.9%.

**Table 3**

| Examples | Mass of Additives/g | Mass of Additives/g | Conversion Rate |
|---|---|---|---|
| 15 | 1.5 | 13.2 | 79.5% |
| 16 | 2.9 | 6.6 | 87.4% |
| 17 | 4.5 | 19.8 | 99.9% |

### Example 18

A reaction device and operation conditions are the same as those of Example 13, The reaction temperature is changed to be 50 °C , after 3 hours of reaction, the conversion rate of perfluoro-2,3-epoxy-2-methyl pentane is 98.3%.

### Example 19

A reaction device and operation conditions are the same as those of Example 17, the reaction temperature is changed to be 50 °C , after 1 hours of reaction, the conversion rate of perfluoro-2,3-epoxy-2-methyl pentane is 99.2%.

### Example 20

900 mL of the aqueous solution of sodium hypochlorite commercially available with 10% of effective chlorine content, 100 mL of acetonitrile and 5.4 mL (4.9 g) of a 30% aqueous solution of trimethylamine are added into a 1500 mL three-neck flask equipped with a reflux condensing tube and a mechanical stirring device, respectively. Stir starts, the temperature is lowered to be 0 °C , 150g of perfluoro-2-methyl-2-pentene is added. After 3 hours of reaction, liquid separation is carried out to obtain 145 g of lower crude products of reaction liquid, yield is 91.8%, the separated reaction liquid is analyzed by gas chromatography, the product is perfluoro-2,3-epoxy-2-methyl pentane, the conversion rate of perfluoro-2-methyl-2-pentene is 77.2%.

### Example 21

900 mL of the aqueous solution of sodium hypochlorite commercially available with 10% of effective chlorine content, 100 mL of acetonitrile and 2.6 mL (1.8 g) of diethylamine are added into a 1500 mL three-neck flask equipped with a reflux condensing tube and a mechanical stirring device, respectively. Stir starts, the temperature is lowered to be 0 °C , 150g of perfluoro-2-methyl-2-pentene is added. After 3 hours of reaction, liquid separation is carried out to obtain 145g of lower crude products of reaction liquid, yield is 91.1%, the separated reaction liquid is analyzed by gas chromatography, the product is perfluoro-2,3-epoxy-2-methyl pentane, the conversion rate of perfluoro-2-methyl-2-pentene is 75.1%.

### Example 22

900 mL of the aqueous solution of sodium hypochlorite commercially available with 10% of effective chlorine content, 100 mL of acetonitrile and 3.5 mL (2.5 g) of triethylamine are added into a 1500 mL three-neck flask equipped with a reflux condensing tube and a mechanical stirring device, respectively. Stir starts, the temperature is lowered to be 0 °C , 150g of perfluoro-2-methyl-2-pentene is added. After 3 hours of reaction, liquid separation is carried out to obtain 144g of lower crude products of reaction liquid, yield is 90.5%, the separated reaction liquid is analyzed by gas chromatography, the product is perfluoro-2,3-epoxy-2-methyl pentane, the conversion rate of perfluoro-2-methyl-2-pentene is 60.0%.

### Example 23

900 mL of the aqueous solution of sodium hypochlorite commercially available with 10% of effective chlorine content, 100 mL of acetonitrile and 1.5g of urea are added into a 1500 mL three-neck flask equipped with a reflux condensing tube and a mechanical stirring device, respectively. Stir starts, the temperature is lowered to be 0 °C, 150g of perfluoro-2-methyl-2-pentene is added. After 3 hours of reaction, liquid separation is carried out to obtain 143g of lower crude products of reaction liquid, yield is 94.9%, the separated reaction liquid is analyzed by gas chromatography, the product is perfluoro-2,3-epoxy-2-methyl pentane, the conversion rate of perfluoro-2-methyl-2-pentene is 83.5%.

### Example 24

900 mL of the aqueous solution of sodium hypochlorite commercially available with 10% of effective chlorine content, 100 mL of acetonitrile and 2.9g of tetramethyl urea are added into a 1500 mL three-neck flask equipped with a reflux condensing tube and a mechanical stirring device, respectively. Stir starts, the temperature is lowered to be 0 °C , 150g of perfluoro-2-methyl-2-pentene is added. After 3 hours of reaction, liquid separation is carried out to obtain 142.5g of lower crude products of reaction liquid, yield is 90.2%, the separated reaction liquid is analyzed by gas chromatography, the product is perfluoro-2,3-epoxy-2-methyl pentane, the conversion rate of perfluoro-2-methyl-2-pentene is 58.5%.

**Table 4**

| Examples | Catalysts | Mass of Additives/g | Volume of Catalysts/mL | Yield | Conversion Rate |
|---|---|---|---|---|---|
| 20 | 30% trimethylamine | 4.9 | 5.4 | 91.8% | 77.2% |
| 21 | Diethylamine | 1.8 | 2.6 | 91.1% | 75.1% |
| 22 | Triethylamine | 2.5 | 3.5 | 90.5% | 60.0% |
| 23 | Urea | 1.5 | - | 94.9% | 83.5% |
| 24 | Tetramethyl urea | 2.9 | - | 90.2% | 58.5% |

### Example 25

900 mL of the aqueous solution of sodium hypochlorite commercially available with 10% of effective chlorine content, 100 mL of acetonitrile and 1.5g of urea are added into a 1500 mL three-neck flask equipped with a reflux condensing tube and a mechanical stirring device, respectively. Stir starts, the temperature is lowered to be 5 °C, 150g of perfluoro-2-methyl-2-pentene is added. After 3 hours of reaction, liquid separation is carried out to obtain 150g of lower crude products of reaction liquid, yield is 95.0%, the separated reaction liquid is analyzed by gas chromatography, the product is perfluoro-2,3-epoxy-2-methyl pentane, the conversion rate of perfluoro-2-methyl-2-pentene is 85.5%.

### Example 26

900 mL of the aqueous solution of sodium hypochlorite commercially available with 10% of effective chlorine content, 100 mL of acetonitrile and 1.5g of urea are added into a 1500 mL three-neck flask equipped with a reflux condensing tube and a mechanical stirring device, respectively. Stir starts, the temperature is lowered to be 10 °C , 150g of perfluoro-2-methyl-2-pentene is added. After 3 hours of reaction, liquid separation is carried out to obtain 151g of lower crude products of reaction liquid, yield is 95.2%, the separated reaction liquid is analyzed by gas chromatography, the product is perfluoro-2,3-epoxy-2-methyl pentane, the conversion rate of perfluoro-2-methyl-2-pentene is 93.7%.

### Example 27

900 mL of the aqueous solution of sodium hypochlorite commercially available with 10% of effective chlorine content, 100 mL of acetonitrile and 1.5g of urea are added into a 1500 mL three-neck flask equipped with a reflux condensing tube and a mechanical stirring device, respectively. Stir starts, the temperature is lowered to be 20 °C , 150g of perfluoro-2-methyl-2-pentene is added. After 3 hours of reaction, liquid separation is carried out to obtain 149g of lower crude products of reaction liquid, yield is 94.4%, the separated reaction liquid is analyzed by gas chromatography, the product is perfluoro-2,3-epoxy-2-methyl pentane, the conversion rate of perfluoro-2-methyl-2-pentene is 90.0%.

### Example 28

900 mL of the aqueous solution of sodium hypochlorite commercially available with 10% of effective chlorine content, 100 mL of acetonitrile and 1.5g of urea are added into a 1500 mL three-neck flask equipped with a reflux condensing tube and a mechanical stirring device, respectively. Stir starts, the temperature is lowered to be 35 °C , 150g of perfluoro-2-methyl-2-pentene is added. After 3 hours of reaction, liquid separation is carried out to obtain 139g of lower crude products of reaction liquid, yield is 88.2%, the separated reaction liquid is analyzed by gas chromatography, the product is perfluoro-2,3-epoxy-2-methyl pentane, the conversion rate of perfluoro-2-methyl-2-pentene is 67.6%.

### Example 29

900 mL of the aqueous solution of sodium hypochlorite commercially available with 10% of effective chlorine content, 100 mL of acetonitrile and 0.6g of urea are added into a 1500 mL three-neck flask equipped with a reflux condensing tube and a mechanical stirring device, respectively. Stir starts, the temperature is lowered to be 10 °C , 150g of perfluoro-2-methyl-2-pentene is added. After 3 hours of reaction, liquid separation is carried out to obtain 151g of lower crude products of reaction liquid, yield is 95.2%, the separated reaction liquid is analyzed by gas chromatography, the product is perfluoro-2,3-epoxy-2-methyl pentane, the conversion rate of perfluoro-2-methyl-2-pentene is 74.9%.

### Example 30

900 mL of the aqueous solution of sodium hypochlorite commercially available with 10% of effective chlorine content, 100 mL of acetonitrile and 3.0g of urea are added into a 1500 mL three-neck flask equipped with a reflux condensing tube and a mechanical stirring device, respectively. Stir starts, the temperature is lowered to be 10 °C , 150g of perfluoro-2-methyl-2-pentene is added. After 3 hours of reaction, liquid separation is carried out to obtain 151g of lower crude products of reaction liquid, yield is 95.2%, the separated reaction liquid is analyzed by gas chromatography, the product is perfluoro-2,3-epoxy-2-methyl pentane, the conversion rate of perfluoro-2-methyl-2-pentene is 99.3%.

### Example 31

900 mL of the aqueous solution of sodium hypochlorite commercially available with 10% of effective chlorine content, 100 mL of acetonitrile and 6.0g of urea are added into a 1500 mL three-neck flask equipped with a reflux condensing tube and a mechanical stirring device, respectively. Stir starts, the temperature is lowered to be 10 °C , 150g of perfluoro-2-methyl-2-pentene is added. After 3 hours of reaction, liquid separation is carried out to obtain 150g of lower crude products of reaction liquid, yield is 95.2%, the separated reaction liquid is analyzed by gas chromatography, the product is perfluoro-2,3-epoxy-2-methyl pentane, the conversion rate of perfluoro-2-methyl-2-pentene is 99.4%.

### Example 32

900 mL of the aqueous solution of sodium hypochlorite commercially available with 10% of effective chlorine content, 100 mL of acetonitrile and 3.0g of urea are added into a 1500 mL three-neck flask equipped with a reflux condensing tube and a mechanical stirring device, respectively. Stir starts, the temperature is lowered to be 10 °C , 150g of perfluoro-2-methyl-2-pentene is added. After 1 hours of reaction, liquid separation is carried out to obtain 151g of lower crude products of reaction liquid, yield is 95.3%, the separated reaction liquid is analyzed by gas chromatography, the product is perfluoro-2,3-epoxy-2-methyl pentane, the conversion rate of perfluoro-2-methyl-2-pentene is 99.3%.

### Comparative Example 1

A reaction device and operation conditions are the same as those of Example 20, but catalyst is not added, the feeding quantity of the aqueous solution of sodium hypochlorite commercially available with 10% of effective chlorine content is changed to be 1400 mL, after 3 hours of reaction, liquid separation is carried out to obtain 132 g of lower crude products of reaction liquid, yield is 83.54%, the separated reaction liquid is analyzed by gas chromatography, the product is perfluoro-2,3-epoxy-2-methyl pentane, the conversion rate of perfluoro-2-methyl-2-pentene is 50.2%.

**Table 5**

| Examples | Reaction Temperature / °C | Mass of Additives/g | Yield | Conversion Rate |
|---|---|---|---|---|
| 25 | 5 | 1.5 | 95.0% | 85.5% |
| 26 | 10 | 1.5 | 95.2% | 93.7% |
| 27 | 20 | 1.5 | 94.4% | 90.0% |
| 28 | 35 | 1.5 | 88.2% | 67.6% |
| 29 | 10 | 0.6 | 95.2% | 74.9% |
| 30 | 10 | 3.0 | 95.2% | 99.3% |
| 31 | 10 | 6.0 | 95.2% | 99.4% |
| 32 | 10 | 3.0 | 95.3% | 99.3% |

### Example 33

100 mL of perfluoro-4-methyl-2-pentene, 1.05 g of sodium fluoride, 6.6 g of 18-crown-6 and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 45.1%.

### Example 34

100 mL of perfluoro-4-methyl-2-pentene, 1.45g of potassium fluoride, 6.6 g of 18-crown-6 and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 81.8%.

### Example 35

100 mL of perfluoro-4-methyl-2-pentene, 3.8g of cesium fluoride, 6.6 g of 18-crown-6 and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 85.0%.

### Example 36

100 mL of perfluoro-4-methyl-2-pentene, 3.8g of cesium fluoride, 5.5g of 15- crown-5 ethers and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 62.1%.

### Example 37

100 mL of perfluoro-4-methyl-2-pentene, 3.8g of cesium fluoride, 3.0g of sulfolane and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 87.3%.

### Example 38

100 mL of perfluoro-4-methyl-2-pentene, 11.4g of cesium fluoride, 9.0g of sulfolane and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 93.7%.

### Example 39

100 mL of perfluoro-4-methyl-2-pentene, 19.0g of cesium fluoride, 15.0g of sulfolane and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 93.7%.

### Example 40

100 mL of perfluoro-4-methyl-2-pentene, 26.6g of cesium fluoride, 21.0g of sulfolane and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 95.2%.

### Example 41

100 mL of perfluoro-4-methyl-2-pentene, 11.4g of cesium fluoride, 18.0g of sulfolane and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 95.1%.

### Example 42

100 mL of perfluoro-4-methyl-2-pentene, 11.4g of cesium fluoride, 27.0g of sulfolane and 100 mL of diglyme are added into a 500 mL three-neck flask equipped with a reflux condensing tube and a mechanical stirring device, respectively. The temperature is raised to be 40 °C after stir starts. After 3 hours of reaction, the reaction product of perfluoro-2-methyl-2-pentene is analyzed by gas chromatography, the conversion rate of Perfluoro-4-methyl-2-pentene is 91.9%.

### Example 43

100 mL of perfluoro-4-methyl-2-pentene, 11.4g of cesium fluoride, 18.0g of sulfolane and 100 mL of diglyme are added into a 500 mL three-neck flask equipped with a reflux condensing tube and a mechanical stirring device, respectively. The temperature is raised to be 55 °C after stir starts. After 3 hours of reaction, the reaction product of perfluoro-2-methyl-2-pentene is analyzed by gas chromatography, the conversion rate of Perfluoro-4-methyl-2-pentene is 98.3%.

### Example 44

100 mL of perfluoro-4-methyl-2-pentene, 11.4g of cesium fluoride, 18.0g of sulfolane and 100 mL of diglyme are added into a 500 mL three-neck flask equipped with a reflux condensing tube and a mechanical stirring device, respectively. The temperature is raised to be 70 °C after stir starts. After 3 hours of reaction, the reaction product of perfluoro-2-methyl-2-pentene is analyzed by gas chromatography, the conversion rate of Perfluoro-4-methyl-2-pentene is 99.5%.

### Example 45

100 mL of perfluoro-4-methyl-2-pentene, 11.4g of cesium fluoride, 18.0g of sulfolane and 100 mL of diglyme are added into a 500 mL three-neck flask equipped with a reflux condensing tube and a mechanical stirring device, respectively. The temperature is raised to be 70 °C after stir starts. After 1.5 hours of reaction, the reaction product of perfluoro-2-methyl-2-pentene is analyzed by gas chromatography, the conversion rate of Perfluoro-4-methyl-2-pentene is 99.5%.

### Example 46

100 mL of perfluoro-4-methyl-2-pentene, 11.4g of cesium fluoride, 18.0g of sulfolane and 100 mL of diglyme are added into a 500 mL three-neck flask equipped with a reflux condensing tube and a mechanical stirring device, respectively. The temperature is raised to be 70 °C after stir starts. After 0.5 hours of reaction, the reaction product of perfluoro-2-methyl-2-pentene is analyzed by gas chromatography, the conversion rate of Perfluoro-4-methyl-2-pentene is 99.5%.

**Table 6**

| Examples | Catalysts | Additives | Solvents | Reaction Temperature /°C | Amount of Catalysts | Amount of Additives/ Mol% | Catalysts /Additives | Reaction Time/H | Conversion Rate |
|---|---|---|---|---|---|---|---|---|---|
| 33 | Sodium Fluoride | 18-Crown-6-Et her | DG | 40 | 5 | 5 | 1 | 3 | 45.1% |
| 34 | Potassium Fluoride | 18-Crown-6-Et her | DG | 40 | 5 | 5 | 1 | 3 | 81.8% |
| 35 | Caesium Fluoride | 18-Crown-6-Et her | DG | 40 | 5 | 5 | 1 | 3 | 85.0% |
| 36 | Caesium Fluoride | 15-Crown-5-Et her | DG | 40 | 5 | 5 | 1 | 3 | 62.1% |
| 37 | Caesium Fluoride | Sulfolane | DG | 40 | 5 | 5 | 1 | 3 | 87.3% |
| 38 | Caesium Fluoride | Sulfolane | DG | 40 | 15 | 15 | 1 | 3 | 93.7% |
| 39 | Caesium Fluoride | Sulfolane | DG | 40 | 25 | 25 | 1 | 3 | 93.7% |
| 40 | Caesium Fluoride | Sulfolane | DG | 40 | 35 | 35 | 1 | 3 | 95.2% |
| 41 | Caesium Fluoride | Sulfolane | DG | 40 | 15 | 30 | 1/2 | 3 | 95.1% |
| 42 | Caesium Fluoride | Sulfolane | DG | 40 | 15 | 45 | 1/3 | 3 | 91.9% |
| 43 | Caesium Fluoride | Sulfolane | DG | 55 | 15 | 30 | 1/2 | 3 | 98.3% |
| 44 | Caesium Fluoride | Sulfolane | DG | 70 | 15 | 30 | 1/2 | 3 | 99.5% |
| 45 | Caesium Fluoride | Sulfolane | DG | 70 | 15 | 30 | 1/2 | 1.5 | 99.5% |
| 46 | Caesium Fluoride | Sulfolane | DG | 70 | 15 | 30 | 1/2 | 0.5 | 99.5% |

## Claims

1. A preparation method for perfluoro-2-methyl-3-pentanone, **characterized in that** in the presence of fluoride salts and ether compounds, perfluoro-2,3-epoxide-2-methyl pentane is converted into perfluoro-2-methyl-3-pentanone by a catalytic rearrangement reaction at the reaction temperature of 10-70 °C;
the fluoride salt is selected from one of or the combination of two or more than three of lithium fluoride, sodium fluoride, potassium fluoride, cesium fluoride and aluminum fluoride;
the ether compound is selected from one of or the combination of two or more than three of diethyl ether, sulfolane, 15-crown-5 and 18-crown-6;
the molar ratio of the ether compound and the fluoride salts is 0.5∼5.0:1;
the molar ratio of the fluoride salts and perfluoro-2,3-epoxy-2-methyl pentane is 0.02∼0.5: 1.

2. A preparation method for perfluoro-2-methyl-3-pentanone according to claim 1, **characterized in that** the fluoride salt is selected from one, two or three of potassium fluoride, cesium fluoride and aluminum fluoride.

3. A preparation method for perfluoro-2-methyl-3-pentanone according to claim 1 or 2, **characterized in that** the ether compound is selected from one, two or three of sulfolane, 15-crown-5 and 18-crown-6.

4. A preparation method for perfluoro-2-methyl-3-pentanone according to claim 1, 2 or 3, **characterized in that** the molar ratio of the ether compound and the fluoride salts is 0.75∼3.0:1.

5. A preparation method for perfluoro-2-methyl-3-pentanone according to claim 1, 2, 3 or 4, **characterized in that** the molar ratio of the fluoride salts and perfluoro-2,3-epoxy-2-methyl pentane is 0.05∼0.35: 1.

6. A preparation method for perfluoro-2-methyl-3-pentanone according to claim 1, 2, 3, 4 or 5, **characterized in that** the perfluoro-2,3-epoxy-2-methyl-pentane is prepared by the reaction of perfluoro-2-methyl-2-pentene and oxidant by using amino compounds as epoxidation catalyst in aprotic solvent and at the reaction temperature of -5 ∼ 35 °C;
the oxidant is selected from one of or the combination of two or three of hypochlorous acid, sodium hypochlorite and calcium hypochlorite;
the amino compound is selected from one of or the combination of two or more three of trimethylamine, diethylamine, triethylamine, urea and tetramethyl urea;
the molar ratio of the oxidant and perfluoro-2-methyl-2-pentene is 1∼2: 1;
the molar ratio of the epoxidation catalyst and perfluoro-2-methyl-2-pentene is 0.02∼0.30: 1.

7. A preparation method for the perfluoro-2-methyl-3-pentanone according to claim 6, **characterized in that** the perfluoro-2-methyl-2-pentene is generated by the isomerization of perfluoro-4-methyl-2-pentene in the aprotic polar solvent under the action of catalyst at the temperature of 40 ∼ 80 °C,
the catalyst consists of procatalyst and cocatalyst,the procatalyst is selected from one of or the combination of two or more than three of lithium fluoride, sodium fluoride, potassium fluoride, cesium fluoride or aluminum fluoride;
the cocatalyst is selected from one of or the combination of two or more than three of diethyl ether, sulfolane, 15-crown-5 or 18- crown-6;
the molar ratio of the procatalyst and cocatalyst is 1∼0.1:1;
the molar ratio of the procatalyst and perfluoro-4-methyl-2-pentene is 0.05∼0.5: 1;
the molar ratio of the cocatalyst and perfluoro-4-methyl-2-pentene is 0.05∼0.5: 1;
the aprotic solvent is selected from one of or the combination of two or more than three of glyme, diglyme, acetonitrile or N, N'- dimethylformamide.

8. A preparation method for perfluoro-2,3-epoxy-2-methyl pentane according to claim 1, **characterized in that** :
perfluoro-2,3-epoxy-2-methyl pentane is prepared by the reaction of perfluoro-2-methyl-2-pentene and the oxidant by using amino compounds as epoxidation catalyst in aprotic solvent and at the reaction temperature of -5 ∼ 35 °C;
the oxidant is selected from one of or the combination of two or three of hypochlorous acid, sodium hypochlorite and calcium hypochlorite;
the amino compound is selected from one of trimethylamine, diethylamine, triethylamine, urea and tetramethyl urea;
the amino compound is selected from one of or the combination of two or more three of trimethylamine, diethylamine, triethylamine, urea and tetramethyl urea;
the molar ratio of the oxidant and perfluoro-2-methyl-2-pentene is 1∼2: 1;
the molar ratio of the epoxidation catalyst and perfluoro-2-methyl-2-pentene is 0.02∼0.30: 1.

9. A preparation method for perfluoro-2,3-epoxy-2-methyl pentane according to claim 8, **characterized in that** :
the perfluoro-2-methyl-2-pentene is generated by the isomerization of perfluoro-4-methyl-2-pentene in aprotic polar solvent under the action of catalyst at the temperature of 40 ∼ 80 °C,
the catalyst consists of procatalyst and cocatalyst,the procatalyst is selected from one of or the combination of two or more than three of lithium fluoride, sodium fluoride, potassium fluoride, cesium fluoride or aluminum fluoride;
the cocatalyst is selected from one of or the combination of two or more than three of diethyl ether, sulfolane, 15-crown-5 or 18- crown-6;
the molar ratio of the procatalyst and the cocatalyst is 1∼0.1:1;
the molar ratio of the procatalyst and perfluoro-4-methyl-2-pentene is 0.05∼0.5: 1;
the molar ratio of the cocatalyst and perfluoro-4-methyl-2-pentene is 0.05∼0.5: 1;
the aprotic solvent is selected from one of or the combination of two or more than three of glyme, diglyme, acetonitrile or N, N'- dimethylformamide.

10. A method for preparing perfluoro-2-methyl-2-pentene according to claim 9, through the catalytic isomerization of the perfluoro-4-methyl-2-pentene, **characterized in that** : the perfluoro-2-methyl-2-pentene is generated by the isomerization of perfluoro-4-methyl-2-pentene in aprotic polar solvent under the action of catalyst at the temperature of 40 ∼ 80 °C,
the catalyst consists of procatalyst and cocatalyst,the procatalyst is selected from one of or the combination of two or more than three of lithium fluoride, sodium fluoride, potassium fluoride, cesium fluoride or aluminum fluoride;
the cocatalyst is selected from one of or the combination of two or more than three of diethyl ether, sulfolane, 15-crown-5 or 18- crown-6;
the molar ratio of the procatalyst and the cocatalyst is 1∼0.1:1;
the molar ratio of the procatalyst and perfluoro-4-methyl-2-pentene is 0.05∼0.5: 1;
the molar ratio of the cocatalyst and perfluoro-4-methyl-2-pentene is 0.05∼0.5: 1;
the aprotic solvent is selected from one of or the combination of two or more than three of glyme, diglyme, acetonitrile or N, N'- dimethylformamide.

## Patentansprüche

1. Herstellungsverfahren für Perfluor-2-methyl-3-pentanon, **dadurch gekennzeichnet, dass** in Gegenwart von Fluoridsalzen und Etherverbindungen Perfluor-2,3-epoxid-2-methylpentan durch eine katalytische Umlagerungsreaktion bei einer Reaktionstemperatur von 10 bis 70 °C zu Perfluor-2-methyl-3-pentanon umgewandelt wird;
das Fluoridsalz ausgewählt ist aus einem oder der Kombination von zwei oder mehr als drei von Lithiumfluorid, Natriumfluorid, Kaliumfluorid, Cäsiumfluorid und Aluminiumfluorid;
die Etherverbindung aus einem oder der Kombination von zwei oder mehr als drei von Diethylether, Sulfolan, 15-Krone-5 und 18-Krone-6 ausgewählt ist;
das Molverhältnis der Etherverbindung und der Fluoridsalze 0,5 bis 5,0 : 1 beträgt;
das Molverhältnis der Fluoridsalze und Perfluor-2,3-epoxy-2-methylpentan 0,02 bis 0,5 : 1 beträgt.

2. Herstellungsverfahren für Perfluor-2-methyl-3-pentanon nach Anspruch 1, **dadurch gekennzeichnet, dass** das Fluoridsalz aus einem, zwei oder drei von Kaliumfluorid, Cäsiumfluorid und Aluminiumfluorid ausgewählt ist.

3. Herstellungsverfahren für Perfluor-2-methyl-3-pentanon nach Anspruch 1 oder 2, **dadurch gekennzeichnet, dass** die Etherverbindung aus einem, zwei oder drei von Sulfolan, 15-Krone-5 und 18-Krone-6 ausgewählt ist.

4. Herstellungsverfahren für Perfluor-2-methyl-3-pentanon nach Anspruch 1, 2 oder 3, **dadurch gekennzeichnet, dass** das Molverhältnis von der Etherverbindung und der Fluoridsalze 0,75 bis 3,0 : 1 beträgt.

5. Herstellungsverfahren für Perfluor-2-methyl-3-pentanon nach Anspruch 1, 2, 3 oder 4, **dadurch gekennzeichnet, dass** das Molverhältnis von den Fluoridsalzen und Perfluor-2,3-epoxy-2-methylpentan 0,05 bis 0,35 : 1 beträgt.

6. Herstellungsverfahren für Perfluor-2-methyl-3-pentanon nach Anspruch 1, 2, 3, 4 oder 5, **dadurch gekennzeichnet, dass** das Perfluor-2,3-epoxy-2-methylpentan durch die Umsetzung von Perfluor-2-methyl-2-penten und Oxidationsmittel unter Verwendung von Aminoverbindungen als Epoxidationskatalysator im aprotischen Lösungsmittel und bei der Reaktionstemperatur von -5 bis 35 °C hergestellt wird;
das Oxidationsmittel ausgewählt ist aus einem oder der Kombination von zwei oder drei von hypochloriger Säure, Natriumhypochlorit und Calciumhypochlorit;
die Aminoverbindung ausgewählt ist aus einem oder der Kombination von zwei oder mehr drei von Trimethylamin, Diethylamin, Triethylamin, Harnstoff und Tetramethylharnstoff;
das Molverhältnis von dem Oxidationsmittel und Perfluor-2-methyl-2-penten 1 bis 2 : 1 beträgt;
das Molverhältnis von dem Epoxidationskatalysator und Perfluor-2-methyl-2-penten 0,02 bis 0,30 : 1 beträgt.

7. Herstellungsverfahren für Perfluor-2-methyl-3-pentanon nach Anspruch 6, **dadurch gekennzeichnet, dass** das Perfluor-2-methyl-2-penten durch die Isomerisierung von Perfluor-4-methyl-2-penten in dem aprotischen polaren Lösungsmittel unter der Einwirkung des Katalysators bei einer Temperatur von 40 bis 80 °C gebildet wird,
der Katalysator aus Prokatalysator und Cokatalysator besteht, wobei der Prokatalysator aus einem oder der Kombination von zwei oder mehr als drei von Lithiumfluorid, Natriumfluorid, Kaliumfluorid, Cäsiumfluorid und Aluminiumfluorid ausgewählt ist;
der Cokatalysator aus einem oder der Kombination von zwei oder mehr als drei von Diethylether, Sulfolan, 15-Krone-5 und 18-Krone-6 ausgewählt ist;
das Molverhältnis von dem Prokatalysator und dem Cokatalysator 1 bis 0,1 : 1 beträgt;
das Molverhältnis von dem Prokatalysator und Perfluor-4-methyl-2-penten 0,05 bis 0,5 : 1 beträgt;
das Molverhältnis von dem Cokatalysator und Perfluor-4-methyl-2-penten 0,05 bis 0,5 : 1 beträgt;
das aprotische Lösungsmittel aus einem oder der Kombination von zwei oder mehr als drei von Glyme, Diglyme, Acetonitril oder N, N'-Dimethylformamid ausgewählt ist.

8. Herstellungsverfahren für Perfluor-2,3-epoxy-2-methylpentan nach Anspruch 1, **dadurch gekennzeichnet, dass**: Perfluor-2,3-epoxy-2-methylpentan durch die Umsetzung von Perfluor-2-methyl-2-penten und dem Oxidationsmittel unter Verwendung von Aminoverbindungen als Epoxidationskatalysator in aprotischem Lösungsmittel und bei der Reaktionstemperatur von -5 bis 35 °C hergestellt wird;
das Oxidationsmittel ausgewählt ist aus einem oder der Kombination von zwei oder drei von hypochloriger Säure, Natriumhypochlorit und Calciumhypochlorit;
die Aminoverbindung ausgewählt ist einem von Trimethylamin, Diethylamin, Triethylamin, Harnstoff und Tetramethylharnstoff;
die Aminoverbindung ausgewählt ist aus einem oder der Kombination von zwei oder mehr drei von Trimethylamin, Diethylamin, Triethylamin, Harnstoff und Tetramethylharnstoff;
das Molverhältnis von dem Oxidationsmittel und Perfluor-2-methyl-2-penten 1 bis 2 : 1 beträgt;
das Molverhältnis von dem Epoxidationskatalysator und Perfluor-2-methyl-2-penten 0,02 bis 0,30 : 1 beträgt.

9. Herstellungsverfahren für Perfluor-2,3-epoxy-2-methylpentan nach Anspruch 8, **dadurch gekennzeichnet, dass**: das Perfluor-2-methyl-2-penten durch die Isomerisierung von Perfluor-4-methyl-2-penten in einem aprotischen polaren Lösungsmittel unter der Einwirkung des Katalysators bei einer Temperatur von 40 bis 80 °C gebildet wird,
der Katalysator aus Prokatalysator und Cokatalysator besteht, wobei der Prokatalysator aus einem oder der Kombination von zwei oder mehr als drei von Lithiumfluorid, Natriumfluorid, Kaliumfluorid, Cäsiumfluorid und Aluminiumfluorid ausgewählt ist;
der Cokatalysator aus einem oder der Kombination von zwei oder mehr als drei von Diethylether, Sulfolan, 15-Krone-5 und 18-Krone-6 ausgewählt ist;
das Molverhältnis von dem Prokatalysator und dem Cokatalysator 1 bis 0,1 : 1 beträgt;
das Molverhältnis von dem Prokatalysator und Perfluor-4-methyl-2-penten 0,05 bis 0,5 : 1 beträgt;
das Molverhältnis von dem Cokatalysator und Perfluor-4-methyl-2-penten 0,05 bis 0,5 : 1 beträgt;
das aprotische Lösungsmittel aus einem oder der Kombination von zwei oder mehr als drei von Glyme, Diglyme, Acetonitril oder N, N'-Dimethylformamid ausgewählt ist.

10. Verfahren zur Herstellung von Perfluor-2-methyl-2-penten nach Anspruch 9 durch die katalytische Isomerisierung des Perfluor-4-methyl-2-pentens, **dadurch gekennzeichnet, dass**: das Perfluor-2-methyl-2-penten durch die Isomerisierung von Perfluor-4-methyl-2-penten in einem aprotischen polaren Lösungsmittel unter der Einwirkung des Katalysators bei einer Temperatur von 40 bis 80 °C gebildet wird,
der Katalysator aus Prokatalysator und Cokatalysator besteht, wobei der Prokatalysator aus einem oder der Kombination von zwei oder mehr als drei von Lithiumfluorid, Natriumfluorid, Kaliumfluorid, Cäsiumfluorid und Aluminiumfluorid ausgewählt ist;
der Cokatalysator aus einem oder der Kombination von zwei oder mehr als drei von Diethylether, Sulfolan, 15-Krone-5 und 18-Krone-6 ausgewählt ist;
das Molverhältnis von dem Prokatalysator und dem Cokatalysator 1 bis 0,1 : 1 beträgt;
das Molverhältnis von dem Prokatalysator und Perfluor-4-methyl-2-penten 0,05 bis 0,5 : 1 beträgt;
das Molverhältnis von dem Cokatalysator und Perfluor-4-methyl-2-penten 0,05 bis 0,5 : 1 beträgt;
das aprotische Lösungsmittel aus einem oder der Kombination von zwei oder mehr als drei von Glyme, Diglyme, Acetonitril oder N, N'-Dimethylformamid ausgewählt ist.

## Revendications

1. Procédé de préparation de perfluoro-2-méthyl-3-pentanone, **caractérisé en ce qu'**en présence de sels de fluorure et de composés éthérés, du perfluoro-2,3-époxy-2-méthylpentane est converti en perfluoro-2-méthyl-3-pentanone par une réaction de réarrangement catalytique à la température de réaction de 10 à 70 °C ;
le sel de fluorure est sélectionné parmi un ou la combinaison de deux ou plus de trois du fluorure de lithium, du fluorure de sodium, du fluorure de potassium, du fluorure de césium et du fluorure d'aluminium ;
le composé éthéré est sélectionné parmi un ou la combinaison de deux ou plus de trois de l'éther diéthylique, du sulfolane, du 15-courrone-5 et du 18-couronne-6 ;
le rapport molaire du composé éthéré et des sels de fluorure est de 0,5 à 5,0 : 1 ;
le rapport molaire des sels de fluorure et du perfluoro-2,3-époxy-2-méthylpentane est de 0,02 à 0,5 : 1.

2. Procédé de préparation de perfluoro-2-méthyl-3-pentanone selon la revendication 1, **caractérisé en ce que** le sel de fluorure est sélectionné parmi un, deux ou trois du fluorure de potassium, du fluorure de césium et du fluorure d'aluminium.

3. Procédé de préparation de perfluoro-2-méthyl-3-pentanone selon la revendication 1 ou 2, **caractérisé en ce que** le composé éthéré est sélectionné parmi un, deux ou trois du sulfolane, du 15-courrone-5 et du 18-couronne-6.

4. Procédé de préparation de perfluoro-2-méthyl-3-pentanone selon la revendication 1, 2 ou 3, **caractérisé en ce que** le rapport molaire du composé éthéré et des sels de fluorure est de 0,75 à 3,0 : 1.

5. Procédé de préparation de perfluoro-2-méthyl-3-pentanone selon la revendication 1, 2, 3 ou 4, **caractérisé en ce que** le rapport molaire des sels de fluorure et du perfluoro-2,3-époxy-2-méthylpentane est de 0,05 à 0,35 : 1.

6. Procédé de préparation de perfluoro-2-méthyl-3-pentanone selon la revendication 1, 2, 3, 4 ou 5, **caractérisé en ce que** le perfluoro-2,3-époxy-2-méthylpentane est préparé par la réaction de perfluoro-2-méthyl-2-pentène et d'un oxydant en utilisant des composés aminés en tant que catalyseur d'époxydation dans un solvant aprotique et à la température de réaction de -5 à 35 °C ;
l'oxydant est sélectionné parmi un ou la combinaison de deux ou trois de l'acide hypochloreux, de l'hypochlorite de sodium et de l'hypochlorite de calcium ;
le composé aminé est sélectionné parmi un ou la combinaison de deux ou plus de trois de la triméthylamine, de la diéthylamine, de la triéthylamine, de l'urée et de la tétraméthylurée ;
le rapport molaire de l'oxydant et du perfluoro-2-méthyl-2-pentène est de 1 à 2 : 1 ;
le rapport molaire du catalyseur d'époxydation et du perfluoro-2-méthyl-2-pentène est de 0,02 à 0,30 : 1.

7. Procédé de préparation de perfluoro-2-méthyl-3-pentanone selon la revendication 6, **caractérisé en ce que** le perfluoro-2-méthyl-2-pentène est produit par l'isomérisation de perfluoro-4-méthyl-2-pentène dans le solvant polaire aprotique sous l'action d'un catalyseur à la température de 40 à 80 °C,
le catalyseur se compose d'un procatalyseur et d'un cocatalyseur, le procatalyseur est sélectionné parmi un ou la combinaison de deux ou plus de trois du fluorure de lithium, du fluorure de sodium, du fluorure de potassium, du fluorure de césium ou du fluorure d'aluminium ;
le cocatalyseur est sélectionné parmi un ou la combinaison de deux ou plus de trois de l'éther diéthylique, du sulfolane, du 15-couronne-5 ou du 18-couronne-6 ;
le rapport molaire du procatalyseur et du cocatalyseur est de 1 à 0,1 : 1 ;
le rapport molaire du procatalyseur et du perfluoro-4-méthyl-2-pentène est de 0,05 à 0,5 : 1 ;
le rapport molaire du cocatalyseur et du perfluoro-4-méthyl-2-pentène est de 0,05 à 0,5 : 1 ;
le solvant aprotique est sélectionné parmi un ou la combinaison de deux ou plus de trois du glyme, du diglyme, de l'acétonitrile ou du N,N'-diméthylformamide.

8. Procédé de préparation de perfluoro-2,3-époxy-2-méthylpentane selon la revendication 1, **caractérisé en ce que** le perfluoro-2,3-époxy-2-méthylpentane est préparé par la réaction du perfluoro-2-méthyl-2-pentène et l'oxydant en utilisant des composés aminés en tant que catalyseur d'époxydation dans un solvant aprotique et à la température de réaction de -5 à 35 °C ;
l'oxydant est sélectionné parmi un ou la combinaison de deux ou trois de l'acide hypochloreux, de l'hypochlorite de sodium et de l'hypochlorite de calcium ;
le composé aminé est sélectionné parmi un ou la combinaison de deux ou trois de la triméthylamine, de la diéthylamine, de la triéthylamine, de l'urée et de la tétraméthylurée ;
le rapport molaire de l'oxydant et du perfluoro-2-méthyl-2-pentène est de 1 à 2 : 1 ;
le rapport molaire du catalyseur d'époxydation et du perfluoro-2-méthyl-2-pentène est de 0,02 à 0,30 : 1.

9. Procédé de préparation de perfluoro-2,3-époxy-2-méthylpentane selon la revendication 8, **caractérisé en ce que** :
le perfluoro-2-méthyl-2-pentène est produit par l'isomérisation de perfluoro-4-méthyl-2-pentène dans un solvant polaire aprotique sous l'action d'un catalyseur à la température de 40 à 80 °C,
le catalyseur se compose d'un procatalyseur et d'un cocatalyseur, le procatalyseur est sélectionné parmi un ou la combinaison de deux ou plus de trois du fluorure de lithium, du fluorure de sodium, du fluorure de potassium, du fluorure de césium ou du fluorure d'aluminium ;
le cocatalyseur est sélectionné parmi un ou la combinaison de deux ou plus de trois de l'éther diéthylique, du sulfolane, du 15-couronne-5 ou du 18-couronne-6 ;
le rapport molaire du procatalyseur et du cocatalyseur est de 1 à 0,1 : 1 ;
le rapport molaire du procatalyseur et du perfluoro-4-méthyl-2-pentène est de 0,05 à 0,5 : 1 ;
le rapport molaire du cocatalyseur et du perfluoro-4-méthyl-2-pentène est de 0,05 à 0,5 : 1 ;
le solvant aprotique est sélectionné parmi un ou la combinaison de deux ou plus de trois du glyme, du diglyme, de l'acétonitrile ou du N,N'-diméthylformamide.

10. Procédé de préparation de perfluoro-2-méthyl-2-pentène selon la revendication 9, par le biais de l'isomérisation catalytique du perfluoro-4-méthyl-2-pentène, **caractérisé en ce que** le perfluoro-2-méthyl-2-pentène est produit par l'isomérisation de perfluoro-4-méthyl-2-pentène dans un solvant polaire aprotique sous l'action d'un catalyseur à la température de 40 à 80 °C,
le catalyseur se compose d'un procatalyseur et d'un cocatalyseur, le procatalyseur est sélectionné parmi un ou la combinaison de deux ou plus de trois du fluorure de lithium, du fluorure de sodium, du fluorure de potassium, du fluorure de césium ou du fluorure d'aluminium ;
le cocatalyseur est sélectionné parmi un ou la combinaison de deux ou plus de trois de l'éther diéthylique, du sulfolane, du 15-couronne-5 ou du 18-couronne-6 ;
le rapport molaire du procatalyseur et du cocatalyseur est de 1 à 0,1 : 1 ;
le rapport molaire du procatalyseur et du perfluoro-4-méthyl-2-pentène est de 0,05 à 0,5 : 1 ;
le rapport molaire du cocatalyseur et du perfluoro-4-méthyl-2-pentène est de 0,05 à 0,5 : 1 ;
le solvant aprotique est sélectionné parmi un ou la combinaison de deux ou plus de trois du glyme, du diglyme, de l'acétonitrile ou du N,N'-diméthylformamide.
